# EUROPEAN PATENT SPECIFICATION

(11) **EP 3 298 408 B1**
(45) Date of publication and mention of the grant of the patent: **05.05.2021**
(21) Application number: 16724366.6
(22) Date of filing: 17.05.2016
(51) Int. Cl.: G01N 33/564, G01N 33/68

(54) **A METHOD FOR DIAGNOSING APS USING DETERMINATION OF ANTI-PAR1 ANTIBODIES**
VERFAHREN ZUR DIAGNOSE VON APS MITTELS BESTIMMUNG VON ANTI-PAR1-ANTIKÖRPERN
PROCÉDÉ DE DIAGNOSTIC DU SYNDROME DES ANTIPHOSPHOLIPIDES PAR LA DÉTERMINATION D'ANTICORPS ANTI-PAR1

(30) Priority: 22.05.2015 EP 15168898
(43) Date of publication of application: 28.03.2018
(73) Proprietor: Celltrend GmbH, 14943 Luckenwalde (DE)
(72) Inventor: HEIDECKE, Harald, 14169 Berlin (DE); SCHULZE-FORSTER, Kai, 12207 Berlin (DE)
(74) Representative: Vossius & Partner Patentanwälte Rechtsanwälte mbB
(86) International application number: PCT/EP2016/061021
(87) International publication number: WO 2016/188801

(56) References cited:
- CHARY LÓPEZ-PEDRERA ET AL: "Differential expression of protease-activated receptors in monocytes from patients with primary antiphospholipid syndrome", ARTHRITIS & RHEUMATISM, vol. 62, no. 3, 25 March 2010 (2010-03-25) , pages 869-877, XP055286239, US ISSN: 0004-3591, DOI: 10.1002/art.27299
- FORD ET AL: "IgG from patients with antiphospholipid syndrome binds to platelets without induction of platelet activation", BRITISH JOURNAL OF HAEMATOLOGY, vol. 102, no. 3, 25 August 1998 (1998-08-25), pages 841-849, XP055286264, GB ISSN: 0007-1048, DOI: 10.1046/j.1365-2141.1998.00841.x

## Description

### Technical Field

The present disclosure relates to the field of medicine, particular to the field of diagnosis of an autoimmune disease. Furthermore, the disclosure relates to the field of detection of PARI antibodies in a sample of a subject to be diagnosed for diagnosis of APS.

### Background

Antiphospholipid syndrome or antiphospholipid antibody syndrome (APS or APLS), or often also Hughes syndrome, is an autoimmune, hypercoagulable state caused by antiphospholipid antibodies. APS provokes blood clots (thrombosis) in both, arteries and veins, as well as pregnancy-related complications such as miscarriage, stillbirth, preterm delivery, and severe preeclampsia. Antiphospholipid syndrome can be primary or secondary. In some cases, APS leads to rapid organ failure due to generalised thrombosis; this is termed "catastrophic antiphospholipid syndrome" (CAPS) and is associated with a high risk of death.

Treatment of antiphospholipid syndrome is usually conducted using anticoagulant medication such as heparin to reduce the risk of further episodes of thrombosis and improve the prognosis of pregnancy.

For diagnosis of APS currently at least one clinical event, i.e. thrombosis or pregnancy complication, and two antibody blood tests spaced at least three months apart that confirm the presence of either lupus anticoagulant, or anti-β2-glycoprotein-I (since β2-glycoprotein-I antibodies are a subset of anti-cardiolipin antibodies, an anti-cardiolipin assay can be performed as a less specific proxy) are needed making the diagnosis laborious and time-consuming.

In terms of antibody blood test, antiphospholipid syndrome is tested for in the laboratory using both liquid phase coagulation assays (lupus anticoagulant) and solid phase ELISA assays (anti-cardiolipin antibodies). Genetic thrombophilia is part of the differential diagnosis of APS and can coexist in some APS patients. Presence of genetic thrombophilia may determine the need for anticoagulation therapy. Thus genetic thrombophilia screening can consist of:
- Further studies for Factor V Leiden variant and the prothrombin G20210A mutation, Factor VIII levels, MTHFR mutation.
- Levels of protein C, free and total protein S, Factor VIII, antithrombin, plasminogen, tissue plasminogen activator (TPA) and plasminogen activator inhibitor-1 (PAI-1)

Further, the testing for antibodies to targets of aPL such as β2 glycoprotein 1 and antiphosphatidyl serine is currently under debate. However, also antibodies directed against non-targets appear to provide an additional tool for diagnosis of APS.

Classification with APS requires evidence of both one or more specific, documented clinical events (either a vascular thrombosis and/or adverse obstetric event) and the confirmed presence of a repeated aPL. The Sapporo APS classification criteria (1998, published in 1999) were replaced by the Sydney criteria in 2006 (Miyakis S, et al. (February 2006). "International consensus statement on an update of the classification criteria for definite antiphospholipid syndrome (APS)". J. Thromb. Haemost. 4 (2): 295-306). Based on the most recent criteria, classification with APS requires one clinical and one laboratory manifestation. The clinical manifestation requires a documented episode of arterial, venous, or small vessel thrombosis - other than superficial venous thrombosis - in any tissue or organ by objective validated criteria with no significant evidence of inflammation in the vessel wall, and/or one or more unexplained deaths of a morphologically normal fetus (documented by ultrasound or direct examination of the foetus) at or beyond the 10th week of gestation and/or 3 or more unexplained consecutive spontaneous abortions before the 10th week of gestation, with maternal anatomic or hormonal abnormalities and paternal and maternal chromosomal causes excluded or at least 1 premature birth of a morphologically normal neonate before the 34th week of gestation due to eclampsia or severe pre-eclampsia according to standard definitions, or recognized features of placental insufficiency. Furthermore two or more laboratory parameters from the following have to be present. Anti-cardiolipin IgG and/or IgM measured by standardized, non-cofactor dependent ELISA on 2 or more occasions, not less than 12 weeks apart; medium or high titre (i.e., > 40 GPL or MPL, or > the 99th percentile), and/or anti-β2 glycoprotein I IgG and/or IgM measured by standardized ELISA on 2 or more occasions, not less than 12 weeks apart; medium or high titre (> the 99th percentile), and/or Lupus anticoagulant detected on 2 occasions not less than 12 weeks apart according to the guidelines of the International Society of Thrombosis and Hemostasis.

APS may be divided into three subtypes: primary (the absence of any comorbidity), secondary (when there is a pre-existing autoimmune condition, most frequently systemic lupus erythematosus, SLE), and catastrophic (when there is simultaneous multi-organ failure with small vessel occlusion). It may be advisable to classify APS into one of the following categories for research purposes 2006 (Miyakis S, et al. (February 2006). "International consensus statement on an update of the classification criteria for definite antiphospholipid syndrome (APS)". J. Thromb. Haemost. 4 (2): 295-306):
I: more than one laboratory criterion present in any combination;
IIa: lupus anticoagulant present alone
IIb: anti-cardiolipin IgG and/or IgM present alone in medium or high titers
IIc: anti-β2 glycoprotein I IgG and/or IgM present alone in a titer greater than 99th percentile

Catastrophic APS diagnosis requires (Asherson RA, Cervera R, de Groot PG, Erkan D, Boffa MC, Piette JC, Khamashta MA, Shoenfeld Y (2003). "Catastrophic antiphospholipid syndrome: international consensus statement on classification criteria and treatment guidelines". Lupus 12 (7): 530-543):
a) Vascular thrombosis in three or more organs or tissues and
b) Development of manifestations simultaneously or in less than a week and
c) Evidence of small vessel thrombosis in at least one organ or tissue and
d) Laboratory confirmation of the presence of aPL.

Even though different tools are used and/or discussed, the diagnosis currently is laborious and time-consuming.

D1 (Lopez-Pedrera et al., 2010; Arthritis & Rheumatism, vol. 62, no. 3, p. 869-877) relates to the differential expression of protease-activated receptors (PARs) in monocytes and their regulation by anticardiolipin antibodies from patients with antiphospholipid syndrome (APS). D2 (Ford et al, 1998; British Journal of Haematology, vol. 102, no. 3, p. 841-849) relates to IgG from patients with antiphospholipid syndrome binding to platelets without induction of platelet activation.

Hence, there is a need for a diagnostic tool and method that allows for improved diagnosis of APS, in particular with high sensitivity and high specificity. The inventors now found that the presence of anti-PAR1 antibodies in samples of the patients provide for both, high sensitivity and high specificity.

### Summary

The invention relates to the embodiments as defined in the claims. In particular, the method as described in the context of the invention relates to an *"in vitro"* method.

The inventors found the presence of auto-antibodies directed against protease-activated receptor 1 in samples of patients suffering from APS. It has furthermore been found that this allows for a diagnostic method with superior specificity and sensitivity as compared to available diagnostic methods. In particular these are superior compared to the ones achieved with other autoimmune diseases. Hence, the present disclosure relates to a method for diagnosis an antiphospholipid syndrome (APS) in a subject wherein, presence or absence of an anti-protease-activated receptor 1 (PARI) antibody is detected in a sample from the subject diagnosed, and wherein the presence of an anti-PAR1 antibody is indicative of the disease.

Furthermore, it may be advisable to compare the levels of anti-PAR1 antibody in the sample of the subject to be diagnosed and a control level, e.g. the levels occurring in samples of subjects without an auto-immune disease, e.g. healthy subjects. Hence, the presence or absence of the anti-PAR1 antibody is detected by the steps of:
(i) determining the level of anti-PAR1 antibodies in a sample of the subject to be diagnosed; and
(ii) comparing the determined level to an anti-PAR1 antibody control level derived from a subject without an autoimmune disease, preferably a reported healthy subject;
wherein an increased level of anti-PAR1 antibody in the sample of the subject to be diagnosed as compared to the anti-PAR1 antibody control level denotes the presence of an anti-PAR1 antibody.

The disclosure also relates to the use of PAR1 protein or an immunogenic peptide thereof for the diagnosis of APS. Also encompassed by the disclosure is the use of a kit in for the diagnosis of APS, wherein the kit comprises PARI protein or an immunogenic peptide thereof.

Treatment of autoimmune diseases often encompasses the removal of antibodies from isolated blood from a patient and thereafter reintroducing the so treated blood into the patient. As the present inventors have demonstrated that anti-PAR1 antibodies are linked to APS it will be acknowledged by the skilled person that the removal of these antibodies is desirable in order to allow treatment of patients. Hence, the invention also relates to a method for the removal of anti-PAR1 antibodies from isolated blood, (i) wherein in a first step the presence or absence of an anti-PAR1 antibody is determined in a blood sample from a subject to be diagnosed for APS; (ii) wherein upon determining the presence of an anti-PAR1 antibody the antibody is removed from isolated blood of the subject.

The invention further relates to an *in vitro* use of a kit for the diagnosis of APS according to the method of the invention, wherein the kit comprises PARI, wherein the PARI is immobilized on a surface. Equally, the present invention relates to an anti-coagulant drug for use in the treatment of APS, wherein the subject to be treated showed presence of anti-PAR1 antibodies in samples.

### Figure Legends

**Figure 1****:** Comparison of levels of anti-PAR1 in samples of healthy subjects and patients suffering from APS. Median levels are shown as thick lines.
**Figure 2****:** ROC-analysis of diagnostic sensitivity and specificity of the presence of anti-PAR1 levels for diagnosis of APS.
**Figure 3****:** Standard curve of the PAR1-Auto-Antibody ELISA

### Detailed Description

The present invention is based on the surprising finding of the inventors that in samples of patients with APS the presence of anti-PAR1 antibodies can be found as compared to healthy subjects. In other words the inventors have found healthy subjects have less detectable antibodies against protease-activated receptor 1 (PARI) in the liquid body samples as compared to patients suffering from APS.

The present invention is based on the finding of that presence or levels of autoimmune-antibodies directed against PARI in subjects have diagnostic and predictive properties. The antibodies to be detected in connection with the present invention are therefore autoantibodies, i.e. those produced by immune system of the subject to be diagnosed or being or to be treated.

In a preferred embodiment APS is selected from the group consisting of primary APS, secondary APS and catastrophic APS. Definitions can be retrieved from the section "Background" as outlined herein above.

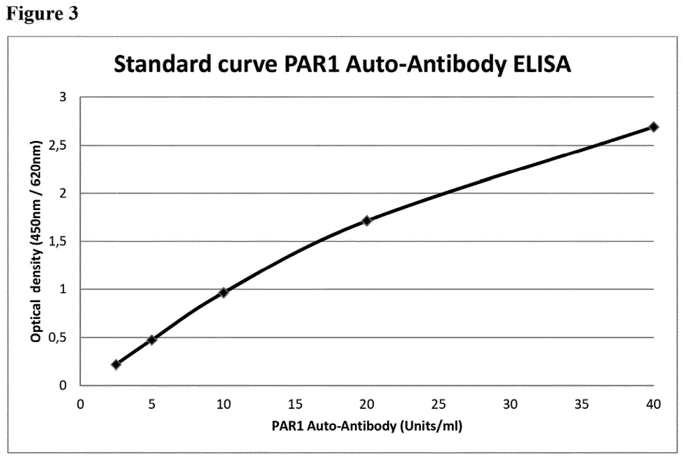

As outlined herein, the presence or absence, or levels, respectively, of anti-PAR1 antibodies in samples of the patient to be diagnosed are determined. Therefore, they may be compared to levels in samples of the control groups as defined herein. However, in one embodiment the levels are compared to fixed values, i.e. thresholds under or over which a certain diagnosis, or prognosis is given. To this end, unit-standards may be applied. The present inventors set out such standard for the PARI using serum samples from systemic sclerosis patients. Systemic sclerosis patients are known to have high levels of autoimmune antibodies in general. Hence, the inventors took a serum sample of a systemic sclerosis patient. However, it will be acknowledged by the skilled person that also other samples may be taken to set a different standard, e.g. samples of healthy subjects, samples of cancer patients. Nevertheless the principle of generating a standard (units) is the same in any case and is exemplified herein using serum samples of systemic sclerosis patients. As described herein, "units/ml", unless specified otherwise, refers to the concentration of antibodies standardised as exemplified herein. Hence, as described herein, 40 units/ml refers to a dilution of 1:100 of a serum sample of systemic sclerosis patients. The serum sample may be derived from a single patient or of a cohort of a plurality of patients, e.g. a cohort of 200 patients suffering from systemic sclerosis. The present inventors found that the concentration of PARI antibodies in samples of systemic sclerosis do not differ by more than about 10 %, showing such standard being reproducible. In one preferred embodiment the standard for the concentrations of the autoimmune antibodies is generated in the following way: a serum sample of a systemic sclerosis patient (or a larger cohort) is diluted (a) 1:100 for standard point 40 Units/ml, (b) 1:200 for standard point 20 Units/ml, (c) 1:400 for standard point 10 Units/ml, (d) 1:800 for standard point 5 Units/ml and (e) 1:1600 for standard point 2.5 Units/ml. These standards are then used for the immunoassay chosen, e.g. ELISA, and then correlated with the respective read-out value, e.g. for ELISA optical density at 450nm/ optical density at 620 nm. A typical standard curve of a PARI auto-antibody ELISA is shown in Figure 3. Nevertheless, the skilled person will readily understand that it may also be possible to standardize the levels of PARI-autoantibodies using different samples.

The invention further pertains to a method for diagnosis of APS, wherein the level of antibodies against protease-activated receptor 1 (PARI) is determined in a sample from a subject to be diagnosed and wherein a level of anti-PAR1 antibodies of more than 4 units/ml is indicative of APS, preferably a level of anti-PAR1 antibodies of more than 5 units/ml, more preferably of more than 7.5 units/ml.

However, it has also been found that ratios may be determined as outlined herein. In such case, it is preferred that the determined levels and the control levels are determined using the same standard, e.g. the one outlined herein or any other standard accessible to the skilled person.
"equal" level as described herein means that the levels differ by not more than ± 10 %, preferably by not more than ± 5 %, more preferably by not more than ± 2 %. "Decreased" or "increased" level as described herein mean that the levels differ by more than 10 %, preferably by more than 15 %, preferably more than 20 %. In terms of ratios, equal preferably relates to ratios between 0.9 fold to 1.1 fold, preferably between 0.95 fold to 1.05 fold, more preferably 0.98 fold to 1.02 fold.

In the context of the present invention, the levels of the anti-PAR1 antibodies a may be analyzed in a number of fashions well known to a person skilled in the art. For example, each assay result obtained may be compared to a "normal" value, or a value indicating a particular disease or outcome. A particular diagnosis/prognosis may depend upon the comparison of each assay result to such a value, which may be referred to as a diagnostic or prognostic "threshold". In certain embodiments, assays for one or more diagnostic or prognostic indicators are correlated to a condition or disease by merely the presence or absence of the indicator(s) in the assay. For example, an assay can be designed so that a positive signal only occurs above a particular threshold concentration of interest, and below which concentration the assay provides no signal above background.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having APS) and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art; *see, e.g.,* Hanley et al. (1982), Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of higher than X may signal that a patient is more likely to suffer from an APS than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value; *see,* e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels for the stratification of subjects into different groups (categories) have to be determined for each particular combination of PAR1-antibodies, disease and/or medication. This can e.g. be done by grouping a reference population of patients according to their level of PAR1-antibodies into certain quantiles, e.g. quartiles, quintiles or even according to suitable percentiles. For each of the quantiles or groups above and below certain percentiles, hazard ratios can be calculated comparing the risk for an adverse outcome, i.e. an "APS", e.g. in terms of presence and absence of APS in patients. In such a scenario, a hazard ratio (HR) above 1 indicates a higher risk for having or acquiring APS. A HR below 1 indicates beneficial effects of a certain treatment in the group of patients. A HR around 1 (e.g. +/- 0.1) indicates no elevated risk but also no benefit from medication for the particular group of patients. By comparison of the HR between certain quantiles of patients with each other and with the HR of the overall population of patients, it is possible to identify those quantiles of patients who have an elevated risk for a disease and those who benefit from medication and thereby stratify subjects according to the present invention.

In some cases presence of APS will affect patients with high levels (e.g. in the fifth quintile) of PAR1-antibodies, while in other cases only patients with low levels of PAR1-antibodies will be affected (e.g. in the first quintile). However, with the above explanations, a skilled person is able to identify those groups of patients having APS. In another embodiment of the invention, the diagnosis for a subject is determined by relating the patient's individual level of marker peptide to certain percentiles (e.g. the 95^{th} or 97.5^{th} percentile) of a healthy population.

The skilled person is able to apply common standards in laboratory diagnosis to the present invention. He may apply any determination method, in particular methods taking into account the level of an analyte. In a preferred embodiment a determined level in said subject to be diagnosed above the 80% percentile of the control levels is attributed to the presence of the antibody in the sample. Consequently, a determined level in said subject to be diagnosed above the 80% percentile of the control levels is attributed to the presence APS in said subject. As will be outlined in greater detail herein below, even increased percentile values may be applied as the cut off. Hence, in one embodiment a determined level in said subject to be diagnosed above the 90% percentile of the control levels is attributed to the presence of the antibody in the sample. Consequently, a determined level in said subject to be diagnosed above the 90% percentile of the control levels is attributed to the presence APS in said subject. In a preferred embodiment a determined level in said subject to be diagnosed above the 95% percentile of the control levels is attributed to the presence of the antibody in the sample. Consequently, a determined level in said subject to be diagnosed above the 95% percentile of the control levels is attributed to the presence APS in said subject. Even higher cut-off values may be applied. The control levels may be derived from subjects not having APS, preferably from healthy subjects or subjects suffering from a depression, more preferably from healthy subjects. The control levels hence are levels derived from a healthy population, i.e. a population of individuals not suffering from an autoimmune disease, preferably not suffering from APS.

Kaplan-Meier estimators may be used for the assessment or prediction of the outcome or risk (e.g. diagnosis) of a patient.

As outlined herein, the presence or absence, or levels, respectively, of anti-PAR1 antibodies in samples of the patient to be diagnosed are determined. Therefore, they may be compared to levels in samples of the control groups as defined herein. In a preferred embodiment the presence or absence of the anti-PAR1 antibody is detected by the following steps: (i) determining the level of anti-PAR1 antibodies in a sample of the subject to be diagnosed; and (ii) comparing the determined level to an anti-PAR1 antibody control level derived from one or more subjects without an autoimmune disease; wherein an increased level of anti-PAR1 antibody in the sample of the subject to be diagnosed as compared to the anti-PAR1 antibody control level denotes the presence of an anti-PAR1 antibody. The subject(s) without an autoimmune disease are preferably confirmed healthy subjects.

From the said above it is evident that the presence of anti-PAR1 antibodies may be determined in terms of being above a certain threshold. As can be derived from Example 2, the mean level of anti-PAR1 antibodies in serum samples of patients suffering from APS is 10.0 units/ml) and in healthy subjects 4.1 units/ml). However, in a preferred embodiment the determination of the presence is performed by determining the ratio of the determined level in the sample of the subject to be diagnosed and the control level. In a preferred embodiment a level in the sample of the subject to be diagnosed of more than 1.1 fold as compared to the control level from subjects without an autoimmune disease, preferably a healthy subject, is indicative of the presence of anti-PAR1 antibodies in the sample of the subject to be diagnosed; preferably a level in the sample of the subject to be diagnosed of more than 1.8 fold as compared to the control level from subjects without an autoimmune disease, preferably a healthy subject, is indicative of the presence anti-PAR1 antibodies in the sample of the subject to be diagnosed; more preferably a level in the sample of the subject to be diagnosed of more than 2 fold as compared to the control level from subjects without an autoimmune disease, preferably a healthy subject, is indicative of the presence of APS in the subject to be diagnosed.

Herein, the sample of the subject to be diagnosed in which the level of anti-PAR1 antibodies is to be determined is preferably a bodily fluid such as whole blood or lymph or fractions of blood such as serum or plasma. Preferably in the context of the present invention the sample is plasma or serum. The inventors found that antibody levels for anti-PAR1 antibodies are similar in serum samples and in plasma. However, in a preferred embodiment the sample in which the level of anti-PAR1 antibodies is to be detected is the same as the sample from which the control levels are derived. That is, if the levels are detected in a plasma sample of the subject to be diagnosed, the determined levels should be compared to control levels derived from plasma samples of the respective control subject. It is also clear from the results provided herewith, that the nature of the so chosen sample does not change the ratio of the levels in cancer patients when comparing them to the control levels measured in the same type of sample.

Where appropriate, the sample may need to be homogenized, or extracted with a solvent prior to use as described herein in order to obtain a liquid sample. A liquid sample hereby may be a solution or suspension. Liquid samples may be subjected to one or more pre-treatments prior to use as described herein. Such pre-treatments include, but are not limited to dilution, filtration, centrifugation, concentration, sedimentation, precipitation, dialysis. Pre-treatments may also include the addition of chemical or biochemical substances to the solution, such as acids, bases, buffers, salts, solvents, reactive dyes, detergents, emulsifiers, chelators.

"Plasma" as described herein is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood) for at least 15 minutes at 2000 to 3000 g.

"Serum" is the liquid fraction of whole blood that is collected after the blood is allowed to clot. When coagulated blood (clotted blood) is centrifuged serum can be obtained as supernatant. It does not contain fibrinogen, although some clotting factors remain.

Hence, the ratio may be directly used to diagnose APC in a subject. Hence, in a preferred embodiment of the present invention a level in the sample of the subject to be diagnosed of more than 1.1 fold as compared to the control level from subjects without an autoimmune disease is indicative of the presence of APS in the subject to be diagnosed; preferably a level in the sample of the subject to be diagnosed of more than 1.8 fold as compared to the control level from subjects without an autoimmune disease is indicative of the presence of APS in the subject to be diagnosed, more preferably a level in the sample of the subject to be diagnosed of more than 2 fold as compared to the control level from subjects without an autoimmune disease is indicative of the presence of APS in the subject to be diagnosed. The control level is preferably derived from one or more healthy subjects.

Furthermore, in the methods of the present invention further parameters of the subject may be considered as well. Such parameters in a multivariate model may include gender, age, clinical and other markers. These predictors can either be measures (as e.g. level of a biomarker) or categorical data (as e.g. response to a previous treatment). The skilled person is aware of the fact that diagnostic markers only give a certain degree of sensitivity and specificity, as also outlined herein. He knows that different further parameters might be considered in order to increase both. Nevertheless, the present invention provides a new and superior marker for diagnosis, prognosis of APS. In the context of the methods of the invention and particularly the immunoassays of the invention, the presence of one or more further diagnostic markers for ABC is detected in a sample from the subject to be diagnosed. For example, in a diagnostic method of the present invention presence of Factor V Leiden variant and the prothrombin G20210A mutation, Factor VIII levels, a MTHFR mutation, levels of protein C, levels of free and total protein S, levels of Factor VIII, levels of antithrombin, levels of plasminogen, levels of tissue plasminogen activator (TPA), levels of plasminogen activator inhibitor-1 (PAI-1), or antibodies directed to targets of aPL, such as β2 glycoprotein 1 and antiphosphatidyl serine may be tested in addition.

When referring to a method for diagnosing it is to be understood that the method preferably relates to the diagnosis of the presence of APS in the subject or to the diagnosis of the risk to acquire APS. Preferably, the method is a method for diagnosis of the presence of APS in the subject to be diagnosed.

The term "anti-PARl", "anti-PAR1 antibody", and "anti-PAR1 autoantibody" as used interchangeably herein, refer to antibodies present in samples of patients suffering from APS, the antibodies specifically binding PARI protein or an immunogenic peptide thereof. Methods for detection of antibodies are known by the skilled person and include immunoassays. Hence, in a preferred embodiment of the present disclosure the anti-PAR1 antibody is detected using an immunoassay comprising the steps of (a) contacting the sample with a protease-activated receptor 1 (PARI) or an antigenic peptide fragment thereof under conditions allowing for the formation of a complex between anti-PAR1 antibodies with PAR1 or the antigenic peptide fragment thereof; (b) detecting the complex.

The antibodies to be detected or determined according to the present invention are directed against PAR1. This means that the antibodies specifically bind PAR1. Specific binding of an antibody normally occurs via binding of a binding site of the antigen, i.e. the epitope. The antibodies of the present invention are those specifically binding to PAR1. This binding may occur via recognition of sequence or structural epitopes. The skilled person is aware of methods of how to determine specific epitopes, e.g. fragments of the antigen PAR1, which are recognized and bound by the antibodies to be determined. Fragments of PAR1 binding to the auto antibodies are called immunogenic fragments. Methods for determining fragments of an antigen binding the antibody are described in several publications (e.g. Gershoni, JM; Roitburd-Berman, A; Siman-Tov, DD; Tarnovitski Freund, N; Weiss, Y (2007), "Epitope mapping: The first step in developing epitope-based vaccines", BioDrugs 21 (3): 145-56; Westwood, MR; Hay, FC (2001), Epitope Mapping: a practical approach, Oxford, Oxfordshire: Oxford University Press. ISBN 0-19-963652-4; Flanagan et al. (2011), "Mapping Epitopes with H/D-Ex Mass Spec", Genetic Engineering and Biotechnology News; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1∗0101, DRB1∗1501, and DRB1∗0401", Clinical and Vaccine Immunology 17 (1): 168-75; Linnebacher, et al. (2012). "Clonality characterization of natural epitope-specific antibodies against the tumor-related antigen topoisomerase IIa by peptide chip and proteome analysis: A pilot study with colorectal carcinoma patient samples", Analytical and Bioanalytical Chemistry, 403 (1): 227-38; Cragg, M. S. (2011) "CD20 antibodies: Doing the time warp", Blood, 118 (2): 219-20; Banik, Soma S. R.; Doranz, Benjamin J. (2010). "Mapping Complex Antibody Epitopes", Genetic Engineering and Biotechnology News, 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR", Journal of the American Chemical Society, 131 (20): 6952-4). In context with the present invention anti-PAR1 antibodies are understood as any immunoglobulin specifically recognizing/binding to PAR1, preferably PARI as defined herein.

As described herein, the terms "PARI" and "PARI-receptor" equally relate to the "protease-activated receptor 1" (also known as "coagulation factor II receptor", "thrombin receptor", "F2R", "TR" and "CF2R"). Protease-activated receptors (PARs) are involved in a number of essential biological processes such as blood clotting, regulation of vascular tone and vascular permeability, motility of the gastrointestinal tract, perception of pain, inflammatory response (including arthritis), angiogenesis, muscle growth and bone cell differentiation and proliferation. PARs are members of the 7-trans-membrane-helix G protein-coupled receptor (GPCR) super family and are activated by cleavage of part of their extracellular domain. They are expressed throughout the body. Particularly high expression occurs in platelets, but also on endothelial cells, myocytes and neurons. Expression of PAR on cells is influenced by the presence of cytokines such as TNFα.

Four different types of PAR receptors have been identified, designated PARI, PAR2, PAR3 and PAR4. PARI and PAR2 are the best studied among the PAR-type receptors and share a sequence homology of 30 %. PAR receptors are activated by the action of serine proteases such as thrombin (PAR 1, 3 and 4) and trypsin (PAR 2). PAR 1 and PAR2 are both activated by Factor Xa, PAR2 is also activated by proteinase III. These proteases cleave the N-terminus of the receptor, which in turn acts as a tethered ligand. In the cleaved state, part of the receptor itself acts as the agonist, causing a physiological response. Most of the PAR-type receptors act through the actions of G-proteins, Raf/Ras activation and calcium signaling to cause cellular actions. Inactivation of PAR receptors is, *inter alia,* mediated by elastases and proteases. A number of agonist and antagonist of PAR have been developed, however, their effects on activation and inhibition of the receptors is highly dependent on the type of cells carrying the respective receptor, the surrounding environment of these cells and ligand concentration. PAR1-deficient mouse embryos were shown to have a 50% intrauterine lethality. In the surviving knockout mice, an increased deposition of extracellular matrix components in the tissues, infiltration of lymphocytes in the lungs and the occurrence of membranoproliferative glomerulonephritis have been observed. PAR2-deficient mice have shown to exhibit a disturbed leukocyte migration and suffer from nephritis, arthritis and pneumonia.

As described herein, the "PARI-receptor" may be present in its natural cellular environment and can be used together with the material associated with the receptor in its natural state as well as in isolated form with respect to its primary, secondary and tertiary structures. The PARI-receptor is well known to those skilled in the art. The receptor is preferably used in isolated form, i.e. essentially free of other proteins, lipids, carbohydrates or other substances naturally associated with the receptor. "Essentially free of" means that the receptor is at least 75%, preferably at least 85%, more preferably at least 95% and especially preferably at least 99% free of other proteins, lipids, carbohydrates or other substances naturally associated with the receptor.

In connection with the present disclosure, the naturally occurring receptor as well as all modifications, mutants or derivatives of the PAR1-receptor can be used. Similarly, a PAR1-receptor produced by means of recombinant techniques, which receptor includes amino acid modifications, such as inversions, deletions, insertions, additions etc. can be used according to the disclosure provided that this part of the essential function of the PARI-receptor is present, namely the capability of binding antibodies. The PARI-receptor being used may also comprise exceptional amino acids and/or modifications of such as alkylation, oxidation, thiol-modification, denaturation, oligomerization and the like. The receptor can also be synthesized by chemical means. According to the disclosure the PARI-receptor particularly can be a protein and/or peptide or a fusion protein, which in addition to other proteins, peptides or fragments thereof, includes the PARI-receptor as a whole or in part. Using conventional methods, peptides or polypeptides of the PARI-receptor which have functionally analogs, analogous properties can be determined by those skilled in the art. For example such polypeptides or peptides have 50-60%, 70% or 80%, preferably 90%, more preferably 95%, and most preferably 98% homology to peptides identified as PARI-receptor, and said homology can be determined, e.g. by means of Smith-Waterman homology search algorithm, using the MPFRCH program (Oxford Molecular), for example.

The term "protein", "receptor" or "polypeptide" of an PAR1-receptor as used in the present disclosure, comprises also molecules differing from the original sequence by deletion(s), insertion(s), substitution(s) and/or other modifications well known in the prior art and/or comprising a fragment of the original amino acid molecule, the PAR1-receptor still exhibiting the properties mentioned herein, preferably being the immunogenic or antigenic peptide according to the disclosure. Such a peptide has preferably at least a length of 100 amino acid residues but may also be shorter, e.g. at least 12, 15, 20 or 25 amino acid residues in length. Also included are allele variants and modifications. Methods of producing the above changes in the amino acid sequence are well known to those skilled in the art and have been described in the standard textbooks of molecular biology, e.g. Sambrook et al (2012), "Molecular Cloning: A Laboratory Manual (Fourth Edition)", Cold Spring Harbor Laboratory, ISBN-10: 1936113414. Those skilled in the art will also be able to determine whether a PARI-receptor, thus, modified still has the properties mentioned above. The amino acid sequence of two isoforms of the PARI receptor is given below and in the attached SEQ ID NO:1 and SEQ ID NO:2. Residues 42 to 425 in SEQ ID NO:1 and SEQ ID NO:2 relate to the amino acid sequence of the mature receptor, while residues 1 to 26 relate to a signal peptide and residues 27 to 41 are removed from the receptor proprotein upon activation. Hence, PARI in the context of the present disclosure preferably relates to the mature PARI receptor corresponding to residues 42 to 425 of SEQ ID NO:1 or SEQ ID NO:2. The receptor may be glycosylated *in vivo.* In the present specification all of the above illustrated modifications of the PARI -receptor will be referred to as "functionally analogous peptides or proteins" in brief. Preferably PARI relates to SEQ ID NO:1.

An "immunogenic peptide" or "antigenic peptide" as used herein interchangeably is a portion of PARI that is recognized (i.e., specifically bound) by the anti-PAR1 antibodies in the sample of the patient to be detected. Such immunogenic peptides generally comprise at least 5 amino acid residues, more preferably at least 10, and still more preferably at least 20 amino acid residues of PAR1. However, they may also comprise at least 30, 40, 50, 60, 70, 80, 90, 100, 110, 120, 130, 140 or 150 amino acid residues. The term "immunogenic peptide" of PAR1 as used in the present disclosure, comprises also molecules differing from the original sequence by deletion(s), insertion(s), substitution(s) and/or other modifications well known in the prior art and/or comprising a fragment of the original amino acid molecule, the receptor still exhibiting the properties mentioned above. Also included are allele variants and modifications. Methods of producing the above changes in the amino acid sequence are well known to those skilled in the art and have been described in the standard textbooks of molecular biology, *see* e.g. Sambrook *et al*., *supra.* Those skilled in the art will also be able to determine whether a PARI immunogenic peptide, thus, modified still has the properties mentioned above. Database entries exist in several well known Databases. When refereeing to the amino acid sequence of PARI any amino acid sequence known is meant, particularly those disclosed in common databases, preferably of human origin. The PARI may be glycosylated *in vivo.* In the present specification all of the above illustrated modifications of the PARI will be referred to as "functionally analogous peptides or proteins" in brief. The immunogenic peptide is preferably a fragment of the sequence of SEQ ID NO:1 or SEQ ID NO:2, preferably a fragment of SEQ ID NO:1.

For the purposes of diagnostic method of the invention PARI may be produced by expression in cells, preferably eukaryotic cells or in cell free, preferably eukaryotic cell free systems. Hence, in the assays and methods of the invention PARI may be present in its natural cellular environment and can be used together with the material associated with the receptor in its natural state as well as in isolated form. Suitable expression systems include Chinese hamster ovary (CHO) cells overexpressing the human PARI. Hence, cell extracts (particularly extracts from CHO cells overexpressing the human PARI) can be used to detect anti-PAR1 antibodies. Based on the weight of the whole receptor in the preparation (e.g. the "extract") to be used according to the invention, the isolated receptor should account for at least 0.5%, preferably at least 5% more preferably at least 25%, and in a particular preferred embodiment at least 50%. The receptor is preferably used in isolated form, i.e. essentially free of other proteins, lipids, carbohydrates or other substances naturally associated with the receptor. "Essentially free of" means that the receptor is at least 75%, preferably at least 85%, more preferably at least 95% and especially preferably at least 99% free of other proteins, lipids, carbohydrates or other substances naturally associated with the receptor.

In the context of the present invention the anti-PAR1 antibody may particularly be selected from the group of IgA-antibody, IgG-antibody and IgM-antibody, preferably an IgG antibody, e.g. IgG1, IgG2, IgG3 and IgG4.

The PAR1 or the antigenic peptide fragment thereof may preferably be immobilized on a surface. The complex may for example preferably be detected using a secondary antibody against the Fc portion of the anti- PARI antibody.

When the anti-PAR1 antibody is an IgG-antibody, the secondary antibody may be an anti-IgG antibody. In a particular embodiment, the subject is a human and
(i) the anti-PAR1 antibody is a IgG1-antibody and the secondary antibody is an anti-human-IgG1 antibody; or
(ii) the anti-PAR1 antibody is a IgG2-antibody and the secondary antibody is an anti-human-IgG2 antibody; or
(iii) the anti-PAR1 antibody is a IgG3-antibody and the secondary antibody is an anti-human-IgG3 antibody; or
(iv) the anti-PAR1 antibody is a IgG4-antibody and the secondary antibody is an anti-human-IgG4 antibody.

The secondary antibody may for example be labeled with a detectable marker, e.g. a peroxidase.

In the method of the present disclosure, the anti-PAR1 antibody is preferably detected in an immunoassay. Suitable immunoassays may be selected from the group of immunoprecipitation, enzyme immunoassay (EIA)), enzyme-linked immunosorbent-assays (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or FACS based assays like Luminex®. Preferably herein the immunoassay is an enzyme linked immunosorbent assay (ELISA).

The immunoassays can be homogenous or heterogeneous assays, competitive and non-competitive assays. In a particularly preferred embodiment, the assay is in the form of a sandwich assay, which is a non-competitive immunoassay, wherein the anti-PAR1 antibody (i.e. the "analyte") to be detected and/or quantified is allowed to bind to an immobilized PARI protein or immunogenic peptide fragment thereof and to a secondary antibody. The PAR1 or fragment thereof (i.e. a peptide), may e.g., be bound to a solid phase, e.g. a bead, a surface of a well or other container, a chip or a strip, and the secondary antibody is an antibody which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person *(*The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb; 10(1):4-10. PMID: 16376134*).* Sandwich immunoassays can for example be designed as one-step assays or as two-step assays.

The detectable label may for example be based on fluorescence or chemiluminescence. The labelling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4' ,5' -dichloro-2' ,7' -dimethodyfluorescein (JOE), N,N,N' ,N' -Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer. Encyclopedia of chemical technology, 4th ed., executive editor. J. I. Kroschwitz; editor. M. Howe-Grant. John Wiley & Sons, 1993, vol. 15, p. 518-562*.* Preferred chemiluminescent dyes are acridiniumesters.

In a particular preferred embodiment the immunoassay is an ELISA.

Furthermore, the disclosure relates to the use of a PAR1 or an immunogenic peptide thereof for the diagnosis of APS. Particulars of the PARI protein and the immunogenic peptide thereof have been outlined herein above and apply also to the uses thereof.

Furthermore, the present disclosure relates to the use of a kit in for the diagnosis of APS, wherein the kit comprises PAR1 or an immunogenic peptide thereof. Particulars of the PARI protein and the immunogenic peptide thereof have been outlined herein above and apply also to the uses thereof. The kit preferably comprises means for detecting antibodies, preferably a secondary antibody binding the Fc portion of the anti-PAR1 antibody to be detected.

The skilled person will understand that controls for comparing the determined levels may be of different nature e.g. depending on the assay used. The kit as disclosed herein may for example comprise one or more controls comprising anti-PAR1 antibodies at the desired control level. Furthermore, the kit may comprise one or more standard solutions each solution comprising anti-PAR1 antibody at different levels, such standard solutions are particularly preferred in cases were a standard curves are to be applied. Exemplary dilutions and levels for such standard solutions are outlined herein above.

The embodiments set out for the immunoassays apply also to the kit used and described herein. The kits as disclosed herein are meant for the detection of autoimmune antibodies in samples of a subject. Hence, in one embodiment they comprise means for the preparation of blood, e.g. for gaining serum or plasma thereof. Furthermore, the kit may comprise control composition and/or standards. The control composition preferably comprises anti-PAR1 antibodies as positive control. Furthermore, the kit may comprise one or a plurality of standard compositions. A standard composition comprises anti-PAR1 antibodies at a defined concentration. As outlined herein, determination of concentration of autoimmune-antibodies may be performed using standard curves. These curves set out which concentration of antibodies in a sample or solution corresponds to what read-out value of the assay used, e.g. optical density or proportion of optical density at different wavelengths (e.g. 450nm/620nm). To this end, the kits as disclosed herein may comprise one or more standard compositions having a defined concentration of anti-PAR1 antibodies, preferably of the kind to be detected in the method. A standard composition of the kits as disclosed herein comprise anti-PAR1 antibodies at concentrations selected from the group consisting of 40 units/ml, 20 units/ml, 10 units/ml, 5 units/ml and 2.5 units/ml. In one embodiment the kit comprises five standard compositions with the recited concentration. In another embodiment the kit comprises one standard composition with the highest concentration of the standard curve or even higher concentrations to allow production of dilutions for different desired concentration, e.g. the kit may comprise a standard compositions comprising 80 units/ml or 40 units/ml of anti-PAR1 antibodies. The other concentrations may be produced at the side of the end user by further dilutions, e.g. in PBS. A dilution buffer may therefore also be comprised in the kits as disclosed herein.

Such kits may further comprise a carrier, package or container that is compartmentalized to receive one or more containers such as vials, tubes, and the like, each of the container(s) comprising one of the separate elements to be used in the method. The kit as disclosed herein will typically comprise the container comprising the elements described above and one or more other containers comprising materials desirable from a commercial and user standpoint, including buffers, diluents, filters, needles, syringes, and package inserts with instructions for use. In addition, a label can be provided on the container to indicate that the composition is used for a specific therapeutic or non-therapeutic application, and can also indicate directions for either *in vivo* or *in vitro* use, such as those described herein. Directions and or other information can also be included on an insert which is included with the kit. The kit preferably comprises means for handling and/or processing a blood sample.

APS is treated by administering an anti-coagulant drug. The terms "anti-coagulant drug" or "anticoagulant" are used synonymously and interchangeably herein. Anticoagulants are a class of drugs that work to prevent the coagulation (clotting) of blood. Such substances occur naturally in leeches and blood-sucking insects. A group of pharmaceuticals called anticoagulants can be used as an injection into human beings as a medication for thrombotic disorders, in particular APS. Oral anticoagulants are also available. Anticoagulants reduce blood clotting which can help prevent coagulation associated diseases. The decision to begin therapeutic anticoagulation often involves the use of multiple bleeding risk predictable outcome tools as non-invasive pre-test stratifications due to the potential for bleeds while on blood thinning agents. Due to the risk of increased bleeding, it is desirable to administer the anti-coagulant drug only if indicated, e.g. only in case an APS is indeed present in a subject.

Hence, the present disclosure also relates to an anti-coagulant drug for use in the treatment of APS, wherein the subject to be treated shows presence of anti-PAR1 antibodies in samples. The presence of anti-PAR1 antibodies in the subject to be treated is detected in samples of the subject. This means that the anti-coagulant drug is only administered to the patient upon detection of anti-PAR1 antibodies in subject to be treated, preferably in a sample as defined herein. The anti-coagulant drug for use according to the invention is administered to the subject upon diagnosis of APS using a method for diagnosis according to the invention. The anti-coagulant drug is administered in case the result of the method for diagnosis according to the invention is indicative of APS.

A number of anticoagulants are available. The traditional ones (warfarin, other coumarins and heparins) are in widespread use. Since the 2000s a number of new agents have been introduced that are collectively referred to as the novel oral anticoagulants (NOACs) or directly acting oral anticoagulants (DOACs). These agents include inhibitors of factor IIa (dabigatran) and factor Xa (rivaroxaban, apixaban and edoxaban) and they have been shown to be as good or possibly better than the coumarins with less serious side effects. The newer anticoagulants (NOACs/DOACs), are more expensive than the traditional ones. In a preferred embodiment the anti-coagulant drug is selected from the group consisting of heparin; low-molecular weight heparin; coumarins such as warfarin, brodifacoum, difenacoum, acenocoumarol, phenprocoumon, atromentin, and phenindione; Novel Oral Anticoagulants (NOACs) such as dabigatran, rivaroxaban, and apixaban; fondaparinux; idraparinux; direct factor Xa inhibitors such as rivaroxaban, apixaban, edoxaban, betrixaban (LY517717; Portola Pharmaceuticals), darexaban (YM150; Astellas), TAK-442 letaxaban (Takeda), and eribaxaban (PD0348292, Pfizer); and direct thrombin inhibitors such as hirudin, lepirudin, bivalirudin dabigatran, and antithrombin. Most preferred is heparin.

Heparin is a biological substance, usually made from pig intestines. It works by activating antithrombin III, which blocks thrombin from clotting blood. Heparin can be used in vivo (by injection), and also in vitro to prevent blood or plasma clotting in or on medical devices. Low molecular weight heparin, a more highly processed product, is useful and has fewer side effects. It may be desirable to remove anti-PAR1 antibodies from isolated blood of a patient diagnosed for the APS. Hence, in one embodiment the invention also relates to a method for the removal of anti-PAR1 antibodies from isolated blood, (i) wherein in a first step the presence or absence of an anti-PAR1 antibody is determined in a blood sample from a subject to be diagnosed for APS; (ii) wherein upon determining the presence of an anti-PAR1 antibody the antibody is removed from isolated blood of the subject.

As outlined above, the invention also relates to a method for removal of anti-PAR1 antibodies from isolated blood. The skilled person will readily acknowledge that the sample in which the presence and/or level of anti-PAR1 antibodies is determined may be the same as the isolated blood from which the anti-PAR1 antibodies are removed. However, the anti-CXCR antibodies are determined in a blood sample of a subject and anti-PAR1 antibodies are than removed from isolated blood of said patient, the isolated blood not being said blood sample. The gist of the invention is the correlation of increased levels of anti-PAR1 antibodies and the presence of APS. Without being bound by theory it is believed that the disease is, *inter alia,* triggered by the immune system of the subject through direction of its activity against the own body or tissues thereof. Hence, the methods as outlined herein above, in particular the method for diagnosing APS, gives the guidance that the immune system is active against own tissues. Hence, it is indicated to remove antibodies from the blood of the patient, e.g. plasmapheresis. Hence, the method for removal of anti-PAR1 antibodies basically relates to two steps, i.e. the determining step and the removal step. It will be acknowledged that the removal step may comprise the removal of more than only anti-PAR1 antibodies, e.g. by the removal of a plurality of immunoglobulins. A specific removal of anti-PAR1 antibodies is not necessary. Hence, in one embodiment of the method for removal of anti-PAR1 antibodies the anti-PAR1 antibodies are removed through removal of a plurality of antibodies comprising anti-PAR1 antibodies, preferably the removal comprises the removal of all IgA-antibodies, and/or all IgG-antibodies and/or all IgM-antibody from said isolated blood, preferably all or a plurality of IgG antibodies, e.g. IgG1, IgG2, IgG3 and IgG4. In one embodiment of the present invention IgG antibodies are removed from isolated blood of a subject upon determination of an anti-PAR1 antibody in a sample of said subject. Removal of a plurality of antibodies is known in the art and also referred to as immunoadsorption.

Removal of anti-PAR1 antibodies may be performed selectively, i.e. only antibodies directed against PARI are removed. However, it may be more feasible to remove total antibodies or subtypes of antibodies from the isolated blood. Hence, in a preferred embodiment of the method for the removal of anti-PAR1 antibodies from isolated blood, the removal step (ii) includes the removal of total antibodies from said isolated blood, serum or plasma. It may be preferred that the removal includes at least a type of antibodies. Hence, in one embodiment the removal step (ii) includes the removal of total IgG, IgM, IgA or IgE antibodies. As outlined herein, the identified anti-PAR1 antibodies in samples of APS patients are of IgG types. Hence, in a particular preferred embodiment step (ii) at least includes the removal of IgG antibodies from said isolated blood, serum or plasma. Means and methods for removing antibodies from isolated blood are commonly known by the skilled person and are commercially available and also referred to as immunapharesis (*see* e.g. Globaffin®, Fresenius Medical Care, Germany).

The present invention is further illustrated by the following non limiting Examples and Figures.

### SEQUENCES

### SEQ ID NO: 1:

Amino acid sequence of the human PAR1 receptor (isoform 1) [SEQ ID NO:1]:

### SEQ ID NO: 2:

Amino acid sequence of the human PAR1 receptor (isoform 2) [SEQ ID NO:2] :

### EXAMPLES

### Example 1:

We measured the anti-PAR1 autoantibody in serum samples using a sandwich ELISA kit (CellTrend GmbH Luckenwalde, Germany). The microtiter 96-well polystyrene plates were coated with chemically synthesized human PARI isoform 1 (SEQ ID NO:1). To maintain the conformational epitopes of the receptor, 1 mM calcium chloride was added to every buffer. Duplicate samples of a 1:100 serum dilution were incubated at 4°C for 2 hours. After washing steps, plates were incubated for 60 minutes with a 1:20.000 dilution of horseradish-peroxidase-labeled goat anti-human IgG (Jackson, USA) used for detection. In order to obtain a standard curve, plates were incubated with test sera from an anti-PAR1 autoantibody positive index patient. The ELISA was validated according to the FDA's "Guidance for industry: Bioanalytical method validation".

To set a standard for the concentrations of the autoimmune antibodies, a standard curve was generated. In detail, a serum sample of a systemic sclerosis patient was diluted (a) 1:100 for standard point 40 Units/ml, (b) 1:200 for standard point 20 Units/ml, (c) 1:400 for standard point 10 Units/ml, (d) 1:800 for standard point 5 Units/ml and (e) 1:1600 for standard point 2.5 Units/ml. Then the optical density was determined using the kit and method of example above. Each standard point was performed in duplicates. The standard curve is depicted in Figure 3.

### Example 2:

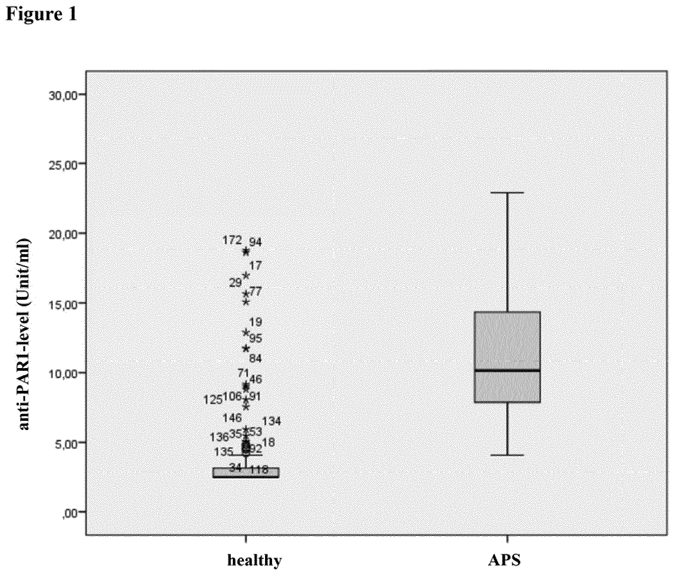

Anti-PAR1 antibody levels in serum samples from 198 healthy donors ("healthy") and 83 patients with APS ("APS") were measured using the kit and method of example 1. The levels were determined in units/mL. Fig. 1 shows the comparison of anti-PAR1 antibody level for case and control subjects. Patient suffering from APS had significantly increased levels (p≤0.05) of anti- PARI antibodies as compared to healthy controls.

The analysis of percentiles revealed the following results:

**Table 1 Percentiles (weighted mean): Anti-PAR1-antibody levels-percentile in healthy donors (n=198) and subjects suffering from APS (n=83).**

| **Percentile** | **5** | **10** | **25** | **50** | **75** | **90** | **95** |
|---|---|---|---|---|---|---|---|
| **healty subjects (units/ml)** | | | | <2,50 | 3,14 | 4,82 | 9,27 |
| **Subjects with APS (units/ml)** | 4,40 | 5,15 | 7,82 | 10,15 | 14,50 | 20,27 | 21,89 |

These data show that a threshold of e.g. 4 unit/ml lies within the 5^{th} percentile of subjects with APS and about 90^{th} of healthy subject, i.e. 95 percent of patients with APS have a higher level than 4 units/ml and 90 percent of healthy subjects have levels below that threshold. This shows that a clear cut-off is possible with the superior method as described herein.

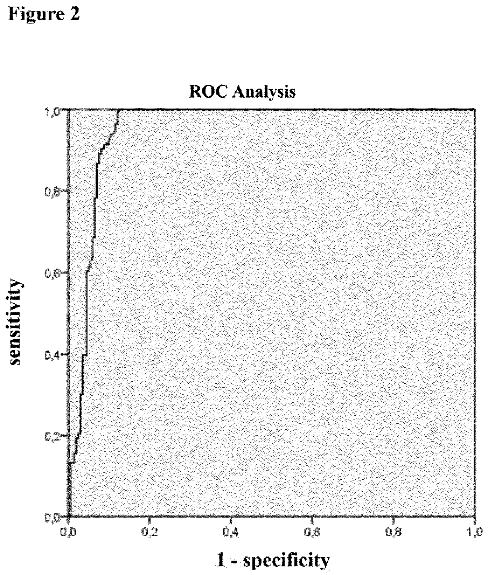

The ROC analysis as depicted in Figure 2 underlines this finding. It shows that the method as described herein provides for a diagnosis which is superior in both, sensitivity and specificity.

### Summary

The results of the present Examples show that anti-PAR1 antibody levels are significantly increased in patients with APS compared to healthy controls. The clear statistical cut-off provides for a method allowing the diagnosis of APS by determining the level of anti-PAR1 antibodies in samples of a subject to be diagnosed.

## Claims

1. An *in vitro* method for diagnosis of antiphospholipid syndrome (APS) in a subject, wherein presence or absence of an anti-protease-activated receptor 1 (PARI) antibody is detected in a sample from the subject to be diagnosed, and wherein the presence of an anti-PAR1 antibody is indicative of the disease, wherein the presence or absence of the anti-PAR1 antibody is detected by the following steps: (i) determining the level of anti-PAR1 antibodies in a sample of the subject to be diagnosed; and (ii) comparing the determined level to an anti-PAR1 antibody control level derived from a subject without an autoimmune disease; wherein an increased level of anti-PAR1 antibody in the sample of the subject to be diagnosed as compared to the anti-PAR1 antibody control level denotes the presence of an anti-PAR1 antibody.

2. The *in vitro* method according to claim 1, wherein a level of anti-PAR1 antibodies in the sample of the subject to be diagnosed above the 80^{th} percentile of control levels from a healthy population is indicative of APS in the subject to be diagnosed.

3. The *in vitro* method according to any one of the preceding claims, wherein the anti-PAR1 antibody is detected using an immunoassay comprising the steps of
(a) contacting the sample with a protease-activated receptor 1 (PARI) under conditions allowing for the formation of a complex between anti-PAR1 antibodies with PARI;
(b) detecting the complex.

4. The *in vitro* method of claim 3, wherein the protease-activated receptor 1 (PARI) is immobilized on a surface.

5. The *in vitro* method according to claim 3 or 4, wherein the protease-activated receptor 1 (PARI) has the sequence of SEQ ID NO:1.

6. The *in vitro* method according to any one of claims 3 to 5, wherein the complex is detected using a secondary antibody against the Fc portion of the anti-PAR1 antibody.

7. The *in vitro* method according to claim 6, wherein the anti-PAR1 antibody is an IgG-antibody and the secondary antibody is an anti-IgG antibody.

8. The *in vitro* method according to claim 6 or 7, wherein the secondary antibody is labeled with a detectable marker.

9. The *in vitro* method according to any one of claims 3 to 8, wherein the immunoassay is selected from the group of immunoprecipitation, enzyme immunoassay (EIA), radioimmunoassay (RIA) or fluorescence immunoassay, a chemilumineszent assay, an agglutination assay, nephelometric assay, turbidimetric assay, a Western blot, a competitive immunoassay, a non-competitive immunoassay, a homogeneous immunoassay a heterogeneous immunoassay and a reporter-assay such as a Luciferase-Assay.

10. The *in vitro* method according to claim 9, wherein the immunoassay is an ELISA

11. The *in vitro* use of a kit for the diagnosis of APS according to the method of any of claims 1 to 10, wherein the kit comprises PARI, wherein the PARI is immobilized on a surface.

12. The *in vitro* use according to claim 11, wherein the kit comprises means for detecting antibodies, preferably a secondary antibody binding the Fc portion of the anti-PAR1 antibody to be detected.

13. The *in vitro* use according to claim 11 or 12, wherein the kit further comprises means for handling and/or processing a blood sample.

14. An anti-coagulant drug for use in the treatment of APS, wherein the subject to be treated showed presence of anti-PAR1 antibodies in samples, wherein the anti-coagulant drug is administered to the subject to be treated upon diagnosis of APS in a method according to any one of claims 1 to 10.

15. The anti-coagulant drug for use according to claim 14, wherein the anti-coagulant drug is heparin.

16. An *in vitro* method for the removal of anti-PAR1 antibodies from isolated blood,
(i) wherein in a first step the presence or absence of an anti-PAR1 antibody is determined in a blood sample from a subject to be diagnosed for APS;
(ii) wherein upon determining the presence of an anti-PAR1 antibody the antibody is removed from isolated blood of the subject.

## Patentansprüche

1. *In vitro*-Verfahren für die Diagnose von Anti-Phospholipid-Syndrom (APS; antiphospholipid syndrome) bei einem Individuum, wobei das Vorliegen oder das Fehlen eines Anti-Protease-aktivierten Rezeptor 1 (PAR1)-Antikörpers in einer Probe von dem zu diagnostizierenden Individuum nachgewiesen wird und wobei das Vorliegen eines Anti-PAR1-Antikörpers auf die Erkrankung hinweist, wobei das Vorliegen oder das Fehlen des Anti-PAR1-Antikörpers durch die folgenden Schritte nachgewiesen wird: (i) Bestimmen des Spiegels von Anti-PAR1-Antikörpern in einer Probe von dem zu diagnostizierenden Individuum; und (ii) Vergleichen des bestimmten Spiegels mit einem Anti-PAR1-Antikörper-Kontrollspiegel, der von einem Individuum ohne eine Autoimmunkrankheit stammt; wobei ein im Vergleich zum Anti-PAR1-Antikörper-Kontrollspiegel erhöhter Spiegel an Anti-PAR1-Antikörpern in der Probe des zu diagnostizierenden Individuums das Vorliegen eines Anti-PAR1-Antikörpers kennzeichnet.

2. *In vitro*-Verfahren nach Anspruch 1, wobei ein Anti-PAR1-Antikörper-Spiegel in der Probe von dem zu diagnostizierenden Individuum oberhalb des 80. Perzentils von Kontrollspiegeln einer gesunden Population auf APS bei dem zu diagnostizierenden Individuum hinweist.

3. *In vitro*-Verfahren nach einem der vorangehenden Ansprüche, wobei der Anti-PAR1-Antikörper unter Verwendung eines Immunassays nachgewiesen wird, der die Schritte umfasst:
(a) Inkontaktbringen der Probe mit einem Protease-aktivierten Rezeptor 1 (PAR1) unter Bedingungen, die die Bildung eines Komplexes aus Anti-PAR1-Antikörpern und PAR1 ermöglichen;
(b) Nachweis des Komplexes.

4. *In vitro*-Verfahren nach Anspruch 3, wobei der Protease-aktivierte Rezeptor 1 (PAR1) auf einer Oberfläche immobilisiert ist.

5. *In vitro*-Verfahren nach Anspruch 3 oder 4, wobei der Protease-aktivierte Rezeptor 1 (PAR1) die Sequenz von SEQ ID NO:1 hat.

6. *In vitro*-Verfahren nach einem der Ansprüche 3 bis 5, wobei der Komplex unter Verwendung eines sekundären Antikörpers gegen den Fc-Teil des Anti-PAR1-Antikörpers nachgewiesen wird.

7. *In vitro*-Verfahren nach Anspruch 6, wobei der Anti-PAR1-Antikörper ein IgG-Antikörper ist und der sekundäre Antikörper ein Anti-IgG-Antikörper ist.

8. *In vitro*-Verfahren nach Anspruch 6 oder 7, wobei der sekundäre Antikörper mit einem nachweisbaren Marker markiert ist.

9. *In vitro*-Verfahren nach einem der Ansprüche 3 bis 8, wobei der Immunassay ausgewählt ist aus der Gruppe aus Immunpräzipitation, Enzym-Immunassay (EIA), Radio-Immunassay (RIA) oder Fluoreszenzimmunassay, einem Chemilumineszenz-Assay, einem Agglutinationsassay, nephelometrischen Assay, turbidimetrischen Assay, einem Western Blot, einem kompetitiven Immunassay, einem nicht-kompetitiven Immunassay, einem homogenen Immunassay, einem heterogenen Immunassay und einem Reporterassay wie zum Beispiel einem Luciferase-Assay.

10. *In vitro*-Verfahren nach Anspruch 9, wobei der Immunassay ein ELISA ist.

11. *In vitro*-Verwendung eines Kits für die Diagnose von APS nach dem Verfahren nach einem der Ansprüche 1 bis 10, wobei der Kit PAR1 umfasst, wobei PAR1 auf einer Oberfläche immobilisiert ist.

12. *In vitro*-Verwendung nach Anspruch 11, wobei der Kit Mittel zum Nachweis von Antikörpern umfasst, bevorzugt einen sekundären Antikörper, der den Fc-Teil des nachzuweisenden Anti-PAR1-Antikörpers bindet.

13. *In vitro*-Verwendung nach Anspruch 11 oder 12, wobei der Kit des Weiteren Mittel zum Behandeln und/oder Prozessieren einer Blutprobe umfasst.

14. Anti-Koagulans-Arzneistoff zur Verwendung bei der Behandlung von APS, wobei das zu behandelnde Individuum das Vorliegen von Anti-PAR1-Antikörpern in Proben gezeigt hat, wobei der Anti-Koagulans-Arzneistoff dem zu behandelnden Individuum nach der Diagnose von APS mit einem Verfahren nach einem der Ansprüche 1 bis 10 verabreicht wird.

15. Anti-Koagulans-Arzneistoff zur Verwendung nach Anspruch 14, wobei der Anti-Koagulans-Arzneistoff Heparin ist.

16. *In vitro*-Verfahren zur Entfernung von Anti-PAR1-Antikörpern aus isoliertem Blut,
(i) wobei in einem ersten Schritt das Vorliegen oder das Fehlen eines Anti-PAR1-Antikörpers in einer Blutprobe von einem in Hinblick auf APS zu diagnostizierenden Individuum bestimmt wird,
(ii) wobei nach Bestimmen des Vorliegens eines Anti-PAR1-Antikörpers der Antikörper aus dem isolierten Blut des Individuums entfernt wird.

## Revendications

1. Procédé *in vitro* pour le diagnostic du syndrome des antiphospholipides (APS) chez un sujet, où la présence ou l'absence d'un anticorps anti-récepteur activé par protéase 1 (PARI) est détectée dans un échantillon provenant du sujet destiné à être diagnostiqué, et où la présence d'un anticorps anti-PAR1 est indicative de la maladie, où la présence ou l'absence de l'anticorps anti-PAR1 est détectée par les étapes suivantes: (i) déterminer le niveau d'anticorps anti-PAR1 dans un échantillon du sujet destiné à être diagnostiqué; et (ii) comparer le niveau déterminé à un niveau témoin d'anticorps anti-PAR1 dérivé d'un sujet sans maladie auto-immune; où un niveau accru d'anticorps anti-PAR1 dans l'échantillon du sujet destiné à être diagnostiqué par rapport au niveau témoin d'anticorps anti-PAR1 indique la présence d'un anticorps anti-PAR1.

2. Procédé *in vitro* selon la revendication 1, où un niveau d'anticorps anti-PAR1 dans l'échantillon du sujet destiné à être diagnostiqué au-dessus du 80^{e} percentile de niveaux témoins provenant d'une population saine est indicatif de l'APS chez le sujet destiné à être diagnostiqué.

3. Procédé *in vitro* selon l'une quelconque des revendications précédentes, où l'anticorps anti-PAR1 est détecté au moyen d'un immunoessai comprenant les étapes de
(a) mettre en contact l'échantillon avec un récepteur activé par protéase 1 (PARI) dans des conditions permettant la formation d'un complexe entre les anticorps anti-PAR1 et PARI;
(b) détecter le complexe.

4. Procédé *in vitro* selon la revendication 3, où le récepteur activé par protéase 1 (PARI) est immobilisé sur une surface.

5. Procédé *in vitro* selon la revendication 3 ou 4, où le récepteur activé par protéase 1 (PARI) a la séquence de SEQ ID NO: 1.

6. Procédé *in vitro* selon l'une quelconque des revendications 3 à 5, où le complexe est détecté au moyen d'un anticorps secondaire contre la partie Fc de l'anticorps anti-PAR1.

7. Procédé *in vitro* selon la revendication 6, où l'anticorps anti-PAR1 est un anticorps IgG et l'anticorps secondaire est un anticorps anti-IgG.

8. Procédé *in vitro* selon la revendication 6 ou 7, où l'anticorps secondaire est marqué avec un marqueur détectable.

9. Procédé *in vitro* selon l'une quelconque des revendications 3 à 8, où l'immunoessai est choisi dans le groupe de l'immunoprécipitation, d'un immunoessai enzymatique (EIA), d'un radioimmunoessai (RIA) ou d'un immunoessai par fluorescence, d'un essai chimiluminescent, d'un essai d'agglutination, d'un essai néphélométrique, d'un essai turbidimétrique, d'un transfert de Western, d'un immunoessai compétitif, d'un immunoessai non compétitif, d'un immunoessai homogène, d'un immunoessai hétérogène et d'un essai à rapporteur tel qu'un essai à la luciférase.

10. Procédé *in vitro* selon la revendication 9, où l'immunoessai est un ELISA.

11. Utilisation *in vitro* d'un kit pour le diagnostic de l'APS selon le procédé de l'une quelconque des revendications 1 à 10, où le kit comprend PARI, où le PARI est immobilisé sur une surface.

12. Utilisation *in vitro* selon la revendication 11, où le kit comprend des moyens pour détecter des anticorps, de préférence un anticorps secondaire liant la partie Fc de l'anticorps anti-PAR1 destiné à être détecté.

13. Utilisation *in vitro* selon la revendication 11 ou 12, où le kit comprend en outre des moyens pour manipuler et/ou traiter un échantillon de sang.

14. Médicament anticoagulant destiné à être utilisé dans le traitement de l'APS, où le sujet destiné à être traité présentait la présence d'anticorps anti-PAR1 dans des échantillons, où le médicament anticoagulant est administré au sujet destiné à être traité lors du diagnostic de l'APS dans un procédé selon l'une quelconque des revendications 1 à 10.

15. Médicament anticoagulant destiné à être utilisé selon la revendication 14, où le médicament anticoagulant est l'héparine.

16. Procédé *in vitro* pour le retrait d'anticorps anti-PAR1 de sang isolé,
(i) où, dans une première étape, la présence ou l'absence d'un anticorps anti-PAR1 est déterminée dans un échantillon de sang provenant d'un sujet destiné à être diagnostiqué pour l'APS;
(ii) où, lors de la détermination de la présence d'un anticorps anti-PAR1, l'anticorps est retiré du sang isolé du sujet.


### SEQUENCE LISTING

<110> CellTrend GmbH
<120> A method for diagnosing APS using determination of anti-PAR1 antibodies
<130> X3311 PCT BLN
<150> EP15 16 8898.3
   <151> 2015-05-22
<160> 2
<170> BiSSAP 1.3
<210> 1
   <211> 425
   <212> PRT
   <213> Homo sapiens
<220>
   <223> Amino acid sequence of the human PAR1 receptor (isoform 1)
<400> 1
   Met Gly Pro Arg Arg Leu Leu Leu Val Ala Ala Cys Phe Ser Leu Cys
   1 5 10 15
   Gly Pro Leu Leu Ser Ala Arg Thr Arg Ala Arg Arg Pro Glu Ser Lys
   20 25 30
   Ala Thr Asn Ala Thr Leu Asp Pro Arg Ser Phe Leu Leu Arg Asn Pro
   35 40 45
   Asn Asp Lys Tyr Glu Pro Phe Trp Glu Asp Glu Glu Lys Asn Glu Ser
   50 55 60
   Gly Leu Thr Glu Tyr Arg Leu Val Ser Ile Asn Lys Ser Ser Pro Leu
   65 70 75 80
   Gln Lys Gln Leu Pro Ala Phe Ile Ser Glu Asp Ala Ser Gly Tyr Leu
   85 90 95
   Thr Ser Ser Trp Leu Thr Leu Phe Val Pro Ser Val Tyr Thr Gly Val
   100 105 110
   Phe Val Val Ser Leu Pro Leu Asn Ile Met Ala Ile Val Val Phe Ile
   115 120 125
   Leu Lys Met Lys Val Lys Lys Pro Ala Val Val Tyr Met Leu His Leu
   130 135 140
   Ala Thr Ala Asp Val Leu Phe Val Ser Val Leu Pro Phe Lys Ile Ser
   145 150 155 160
   Tyr Tyr Phe Ser Gly Ser Asp Trp Gln Phe Gly Ser Glu Leu Cys Arg
   165 170 175
   Phe Val Thr Ala Ala Phe Tyr Cys Asn Met Tyr Ala Ser Ile Leu Leu
   180 185 190
   Met Thr Val Ile Ser Ile Asp Arg Phe Leu Ala Val Val Tyr Pro Met
   195 200 205
   Gln Ser Leu Ser Trp Arg Thr Leu Gly Arg Ala Ser Phe Thr Cys Leu
   210 215 220
   Ala Ile Trp Ala Leu Ala Ile Ala Gly Val Val Pro Leu Leu Leu Lys
   225 230 235 240
   Glu Gln Thr Ile Gln Val Pro Gly Leu Asn Ile Thr Thr Cys His Asp
   245 250 255
   Val Leu Asn Glu Thr Leu Leu Glu Gly Tyr Tyr Ala Tyr Tyr Phe Ser
   260 265 270
   Ala Phe Ser Ala Val Phe Phe Phe Val Pro Leu Ile Ile Ser Thr Val
   275 280 285
   Cys Tyr Val Ser Ile Ile Arg Cys Leu Ser Ser Ser Ala Val Ala Asn
   290 295 300
   Arg Ser Lys Lys Ser Arg Ala Leu Phe Leu Ser Ala Ala Val Phe Cys
   305 310 315 320
   Ile Phe Ile Ile Cys Phe Gly Pro Thr Asn Val Leu Leu Ile Val His
   325 330 335
   Tyr Ser Phe Leu Ser His Thr Ser Thr Thr Glu Ala Ala Tyr Phe Ala
   340 345 350
   Tyr Leu Leu Cys Val Cys Val Ser Ser Ile Ser Cys Cys Ile Asp Pro
   355 360 365
   Leu Ile Tyr Tyr Tyr Ala Ser Ser Glu Cys Gln Arg Tyr Val Tyr Ser
   370 375 380
   Ile Leu Cys Cys Lys Glu Ser Ser Asp Pro Ser Ser Tyr Asn Ser Ser
   385 390 395 400
   Gly Gln Leu Met Ala Ser Lys Met Asp Thr Cys Ser Ser Asn Leu Asn
   405 410 415
   Asn Ser Ile Tyr Lys Lys Leu Leu Thr
   420 425
<210> 2
   <211> 425
   <212> PRT
   <213> Homo sapiens
<220>
   <223> Amino acid sequence of the human PAR1 receptor (isoform 2)
<400> 2
   Met Gly Pro Arg Arg Leu Leu Leu Val Ala Ala Cys Phe Ser Leu Cys
   1               5                   10                  15
   Gly Pro Leu Leu Ser Ala Arg Thr Arg Ala Arg Arg Pro Glu Ser Lys
               20                  25                  30
   Ala Thr Asn Ala Thr Leu Asp Pro Arg Ser Phe Leu Leu Arg Asn Pro
           35                  40                  45
   Asn Asp Lys Tyr Glu Pro Phe Trp Glu Asp Glu Glu Lys Asn Glu Ser
       50                  55                  60
   Gly Leu Thr Glu Tyr Arg Leu Val Ser Ile Asn Lys Ser Ser Pro Leu
   65                  70                  75                  80
   Gln Lys Gln Leu Pro Ala Phe Ile Ser Glu Asp Ala Ser Gly Tyr Leu
                   85                  90                  95
   Thr Ser Ser Trp Leu Thr Leu Phe Val Pro Ser Val Tyr Thr Gly Val
               100                 105                 110
   Phe Val Val Ser Leu Pro Leu Asn Ile Met Ala Ile Val Val Phe Ile
           115                 120                 125
   Leu Lys Met Lys Val Lys Lys Pro Ala Val Val Tyr Met Leu His Leu
       130                 135                 140
   Ala Thr Ala Asp Val Leu Phe Val Ser Val Leu Pro Phe Lys Ile Ser
   145                 150                 155                 160
   Tyr Tyr Phe Ser Gly Ser Asp Trp Gln Phe Gly Ser Glu Leu Cys Arg
                   165                 170                 175
   Phe Val Thr Ala Ala Phe Tyr Cys Asn Met Tyr Ala Ser Ile Leu Leu
               180                 185                 190
   Met Thr Val Ile Ser Ile Asp Arg Phe Leu Ala Val Val Tyr Pro Met
           195                 200                 205
   Gln Ser Leu Ser Trp Arg Thr Leu Gly Arg Ala Ser Phe Thr Cys Leu
       210                 215                 220
   Ala Ile Trp Ala Leu Ala Ile Ala Gly Val Val Pro Leu Leu Leu Lys
   225                 230                 235                 240
   Glu Gln Thr Ile Gln Val Pro Gly Leu Asn Ile Thr Thr Cys His Asp
                   245                 250                 255
   Val Leu Asn Glu Thr Leu Leu Glu Gly Tyr Tyr Ala Tyr Tyr Phe Ser
               260                 265                 270
   Ala Phe Ser Ala Val Phe Phe Phe Val Pro Leu Ile Ile Ser Thr Val
           275                 280                 285
   Cys Tyr Val Ser Ile Ile Arg Cys Leu Ser Ser Ser Ala Val Ala Asn
       290                 295                 300
   Arg Ser Lys Lys Ser Arg Ala Leu Phe Leu Ser Ala Ala Val Phe Cys
   305                 310                 315                 320
   Ile Phe Ile Ile Cys Phe Gly Pro Thr Asn Val Leu Leu Ile Ala His
                   325                 330                 335
   Tyr Ser Phe Leu Ser His Thr Ser Thr Thr Glu Ala Ala Tyr Phe Ala
               340                 345                 350
   Tyr Leu Leu Cys Val Cys Val Ser Ser Ile Ser Cys Cys Ile Asp Pro
           355                 360                 365
   Leu Ile Tyr Tyr Tyr Ala Ser Ser Glu Cys Gln Arg Tyr Val Tyr Ser
       370                 375                 380
   Ile Leu Cys Cys Lys Glu Ser Ser Asp Pro Ser Ser Tyr Asn Ser Ser
   385                 390                 395                 400
   Gly Gln Leu Met Ala Ser Lys Met Asp Thr Cys Ser Ser Asn Leu Asn
                   405                 410                 415
   Asn Ser Ile Tyr Lys Lys Leu Leu Thr
               420                 425